(19) Europäisches Patentamt
European Patent Office
Office européen des brevets

(11) Veröffentlichungsnummer: **0 574 666 A1**

(12) **EUROPÄISCHE PATENTANMELDUNG**

(21) Anmeldenummer: **93106039.6**

(51) Int. Cl.5: **C07D 211/94**

(22) Anmeldetag: **14.04.93**

(30) Priorität: **13.06.92 DE 4219471**

(43) Veröffentlichungstag der Anmeldung:
**22.12.93 Patentblatt 93/51**

(84) Benannte Vertragsstaaten:
**BE CH DE FR GB IT LI NL**

(71) Anmelder: **HÜLS AKTIENGESELLSCHAFT**

**D-45764 Marl(DE)**

(72) Erfinder: **Kaufhold, Manfred, Dr.**
**Jasminweg 20**
**W-4370 Marl(DE)**
Erfinder: **Büschken, Wilfried, Dr.**
**Rosenkamp 10**
**W-4358 Haltern 6(DE)**
Erfinder: **Bickert, Peter, Dr.**
**Eupener Weg 11 A**
**W-4400 Münster(DE)**

(54) **N-Oxyl-Derivate des 2,2,6,6-Tetramethylpiperidins und deren Herstellung.**

(57) Die Erfindung betrifft neue N-Oxyl-Derivate des 2,2,6,6-Tetramethylpiperidins der allgemeinen Formel:

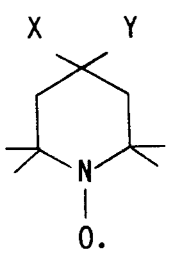

I

1. Ketale, worin

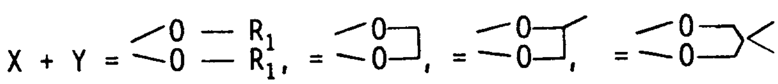

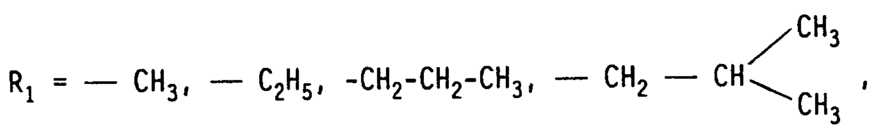

— $CH_2$-$CH_2$-$CH_2$-$CH_3$,

2. Ether, worin

Y = — H, X = $OR_2$

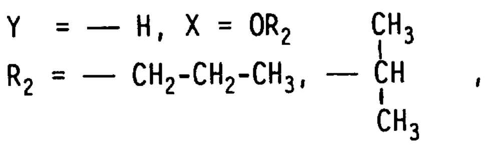

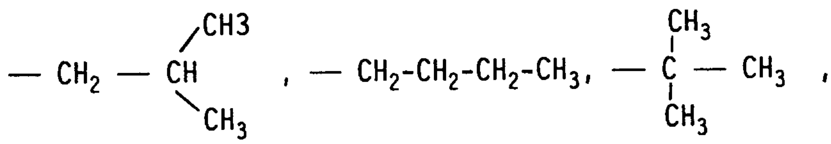

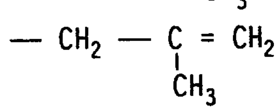

und deren Herstellung.

EP 0 574 666 A1

Die Erfindung betrifft neue N-Oxyl-Derivate des 2,2,6,6-Tetramethylpiperidins der allgemeinen Formel:

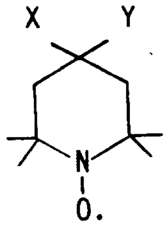

I

1. Ketale, worin

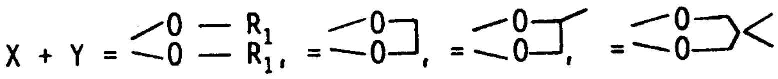

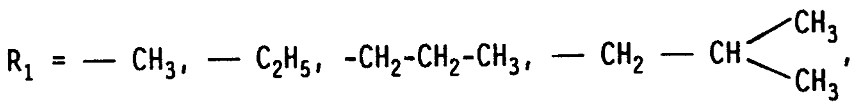

$-CH_2-CH_2-CH_2-CH_3$,

2. Ether, worin

$Y = -H$, $X = OR_2$

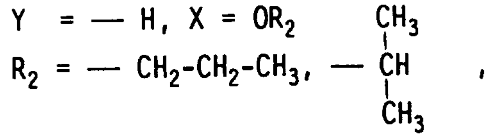

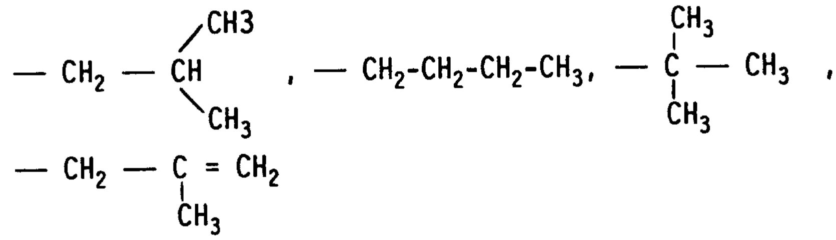

Bekannte N-Oxyl-Derivate des 2,2,6,6-Tetramethylpiperidins (TEMP) entsprechen der Formel A und werden durch Oxidation von 2,2,6,6-Tetramethylpiperidin der Formel B erhalten:

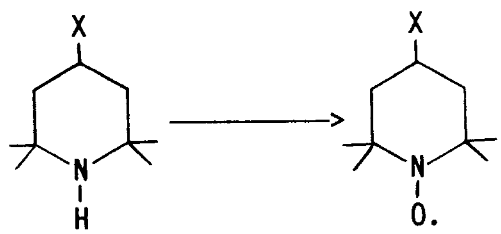

B A

,

X bedeutet darin u. a. H, Hal, -O-Alkyl, -COO-Alkyl.

Einige der literaturbekannten N-Oxyle des 2,2,6,6-Tetramethylpiperidins haben auch technische Bedeutung erlangt, wie z. B. als Stabilisatoren für polymere Werkstoffe, als Lichtschutzmittel usw. Das Besondere an ihnen ist, daß es sich chemisch um stabile Radikale handelt. Darum haben sie zunehmend auch als Redox-Katalysatoren an Bedeutung gewonnen.

Bei diesen Oxidationen arbeitet man häufig in zweiphasigen Systemen, wobei die zu oxidierende Substanz die organische Phase bildet, während das Oxidationsmittel, wie z. B. Natriumhypochlorit, in der wäßrigen Phase vorliegt. Die Löslichkeiten beider Phasen ineinander sind meistens sehr gering.
Deshalb spielt die Löslichkeit des Katalysators eine ganz besondere Rolle. Er transportiert sozusagen die Oxidationsenergie von der wäßrigen in die organische Phase. Das kann er nur leisten, wenn er in beiden Phasen eine gewisse Löslichkeit hat. Die Substituenten X und Y dürfen also nicht zu hydrophil und nicht zu hydrophob sein. So ist z. B. 4-Hydroxi-2,2,6,6-tetramethylpiperidin wegen seiner guten Löslichkeit in Wasser in vielen Fällen kein geeigneter Katalysator. Dagegen benötigt man beim Einsatz des unsubstituierten N-Oxyls des 2,2,6,6-Tetramethylpiperidins wegen seiner geringen Polarität zusätzliche Phasentransfer-Katalysatoren in größerer Menge.

Eine weitere Bedingung, welche die Redox-Katalysatoren erfüllen müssen, ist eine hinreichende Stabilität auch in stark basischem Medium. Diese Bedingung wird von vielen bekannten N-Oxylen, wie z. B. Estern, nicht hinreichend erfüllt.

Es besteht darum ein Bedarf an neuen N-Oxyl-Derivaten des 2,2,6,6-Tetramethylpiperidins, die diesen Anforderungen gerecht werden, und es war daher Aufgabe der Erfindung, solche zu entwickeln.

Diese Aufgabe wurde gelöst mit Hilfe von bisher unbekannten längerkettigen Ethern und Acetalen dieses N-Oxyls.

Gegenstand der Erfindung sind daher N-Oxyl-Derivate des 2,2,6,6-Tetramethylpiperidins der allgemeinen Formel, beinhaltend:

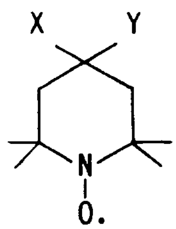

I

1. Ketale, worin

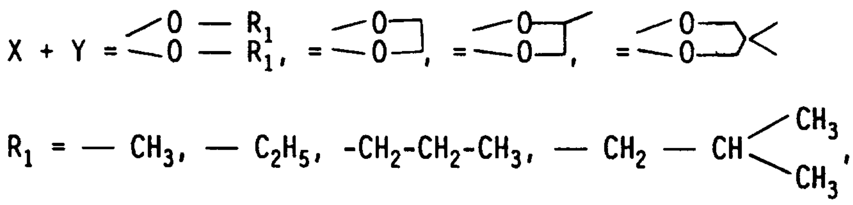

— $CH_2-CH_2-CH_2-CH_3$,

2. Ether, worin

Y = — H, X = $OR_2$

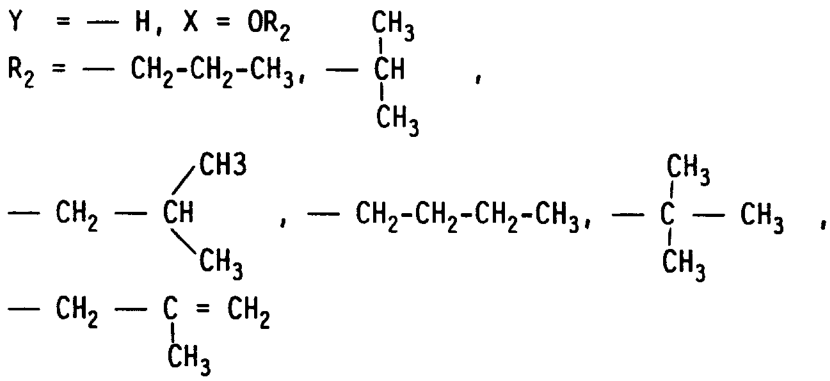

Die Herstellung der Ausgangssubstanzen, d. h. der Derivate des 2,2,6,6-Tetramethylpiperidins (TEMP), mit der Strukturformel II erfolgt nach literaturbekannten Verfahren. So erhält man z. B. die Ketale durch Ketalisierung von Triacetonamin mit Alkoholen und Glykolen nach GB-PS 1 336 403 und die Ether durch übliche Alkylierungsreaktionen.

Die Oxidation der Derivate des TEMP zu den N-Oxylen erfolgt mit Wasserstoffsuperoxid unter der Katalyse von zweiwertigen Metallsalzen.

Es wurde gefunden, daß TEMP und seine Derivate durch Wasserstoffsuperoxid unter der Katalyse von zweiwertigen Metallsalzen nahezu quantitativ mit sehr hoher Selektivität von über 90 %, in einigen Fällen sogar über 97 %, oxidiert werden, wobei die Konzentrationen des Metallsalzes extrem niedrig sind. Als zweiwertige Metallsalze können Verbindungen des Magnesiums, Calciums, der anderen Erdalkalimetalle, wie Barium und Strontium, und Verbindungen des Zinks eingesetzt werden, z. B. in Form der Chloride, Sulfate, Nitrate, Phosphate, Hydroxide usw. Voraussetzung ist, daß die Salze wasserlöslich sind und in dem Reaktionsgemisch in Ionenform vorliegen. Selbstverständlich können auch alle Verbindungen eingesetzt werden, die mit dem Reaktionsgemisch reagieren und dann in Ionen dissoziieren, wie z. B. Metallalkoholate oder Grignard-Verbindungen oder die Metalle selbst usw. Die Salze können als reiner Stoff, in Form einer Lösung und auch als Gemische eingesetzt werden.

Weiterer Gegenstand der Erfindung ist daher ein Verfahren zur Herstellung der N-Oxyle nach Anspruch 1, welches dadurch gekennzeichnet ist, daß die Oxidation des entsprechenden Derivates des 2,2,6,6-Tetramethylpiperidins der allgemeinen Formel II

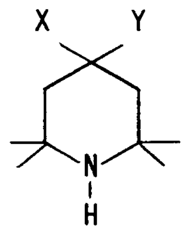

II

mittels Wasserstoffsuperoxid unter der Katalyse von zweiwertigen Metallsalzen erfolgt.

Entscheidend ist die extrem niedrige Konzentration, die völlig ausreicht, um die guten Ausbeuten zu erzielen:

Das molare Verhältnis von Einsatzstoff zum Metallsalz beträgt $10^5$ : 1 bis 10 : 1, vorzugsweise $10^5$ : 1 bis $10^2$ : 1, insbesondere $10^4$ : 1.

Wegen dieser geringen Konzentration ist der Katalysatorverbrauch sehr gering. Außerdem ist es vorteilhaft, daß es äußerst billige Chemikalien sind. Ein weiterer Vorteil ist, daß es sich um Chemikalien handelt, die die Umwelt nicht belasten, da Verbindungen, z. B. des Magnesiums und Calciums in der Natur weit verbreitet sind, so daß Spuren dieser Verbindungen in Abfallprodukten nicht zu Umweltschutzproblemen führen können.

Die Umsetzung wird in wäßriger Lösung oder in Suspension durchgeführt. Ob ein Lösungsmittel zusätzlich eingesetzt wird oder nicht, richtet sich nach der Polarität bzw. nach der Löslichkeit der zu oxidierenden Verbindung in Wasser. Wird z. B. 4-Hydroxi-TEMP ($X = OR_1$, $R_1 = H$), eine sehr polare Verbindung eingesetzt, so erübrigt sich jedes weitere Lösungsmittel.

Die Aufarbeitung erfolgt nach den üblichen Methoden und ist in den meisten Fällen sehr einfach, da die Ausgangsverbindung fast vollständig umgesetzt wird. Beispielsweise wird bei der Oxidation des 4-Hydroxi-TEMP, bei der kein Lösungsmittel benötigt wird, einfach so aufgearbeitet, daß Wasser im Vakuum abdestilliert wird. Als Rückstand verbleibt das entsprechende N-Oxyl mit einer Reinheit von über 99 %. Da die Katalysatorkonzentration im Endprodukt kleiner als 100 ppm beträgt, erübrigt sich für die meisten Anwendungszwecke eine Abtrennung. Selbstverständlich kann das Reaktionsprodukt beispielsweise durch Umkristallisieren gereinigt und dabei der Katalysator entfernt werden.

Die folgenden Beispiele zeigen neue N-Oxyl-Verbindungen gemäß der Formel I, wobei von den TEMP-Derivaten der Formel II ausgegangen wird:

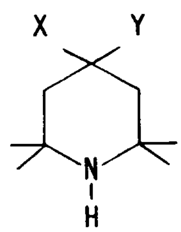

II

| Derivat-Nr. | Bezeichnung | |
|---|---|---|
| 1. | Ethylenglykolketal: | X und Y = 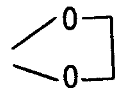 |
| 2. | Methylether: | Y = H, X = $-OCH_3$ |
| 3. | Ethylether: | Y = H, X = $-O-C_2H_5$ |
| 4. | n-Butylether: | Y = H, X = $-O-C_4H_9$ |
| 5. | Methallylether: | Y = H, X = $-O-CH_2-\overset{CH_3}{\overset{\vert}{C}}=CH_2$ |

Beispiel 1

Man benutzt eine Glasapparatur, die aus einem Dreihalskolben mit Rührer, Thermometer und Tropftrichter besteht.

Man setzt ein:
31,0 g (= 0,2 Mol) Ethylenglykolketal, Nr. 1
25 g Wasser
5,8 mg (= 0,1 mMol) Magnesiumhydroxid
und erwärmt das Gemisch unter Rühren auf 70 °C. Dann wird bei dieser Temperatur in einer Stunde 45,3 g (= 0,4 Mol) 30 %ige wäßrige Wasserstoffsuperoxidlösung zugetropft und 6 Stunden bei 70 °C gerührt. Danach werden 90 % des Wassers bei 40 hPa abdestilliert. Zur Verbesserung des Umsatzes werden zu dem Destillationsrückstand nochmals 5,8 mg (= 0,1 mMol) Magnesiumhydroxid gegeben, bei 70 °C 45,3 g (= 0,4 Mol) 30 %ige wäßrige Wasserstoffsuperoxidlösung zugetropft und weitere 5 Stunden bei 70 °C gerührt. Dann wird Wasser bei 40 hPa abdestilliert. Der Destillationsrückstand, 39,8 g, enthält das gewünschte N-Oxyl in 96 %iger Reinheit. Das entspricht einer Ausbeute von ca. 88 % der Theorie.

Beispiele 2 bis 5

Man benutzt eine Glasapparatur, die aus einem Dreihalskolben mit Rührer, Thermometer, Tropftrichter und Rückflußkühler besteht.
Man setzt ein:
0,4 Mol TEMP-Derivat s. Tabelle
100 ml Methanol
8,14 mg (= 0,04 mMol) Magnesiumchlorid mit 6 Kristallwasser gelöst in 2 ml Wasser
Das TEMP-Derivat, Methanol und die Magnesiumchloridlösung werden zusammengegeben und auf 65 °C erwärmt.
Dann werden innerhalb von 90 Minuten
90,6 g (= 0,8 Mol) wäßrige Wasserstoffsuperoxidlösung, 30 %ig,
zugetropft und danach 7 Stunden bei 65 °C gerührt. Die Reaktionsprodukte werden mittels gaschromatographischer Analyse untersucht und der Umsatz bestimmt, s. Tabelle.

Zur Reinigung der N-Oxyle werden 10 ml 10 %ige Schwefelsäure zugesetzt und das Reaktionsgemisch viermal mit je 50 ml Cyclohexan extrahiert. Das Cyclohexan wird abdestilliert und der Destillationsrückstand, das gewünschte N-Oxyl, analysiert.

Die Ergebnisse zeigt die folgende Tabelle:

| Einsatzprodukt TEMP-Derivat Nr. | Endprodukt, N-Oxyl Reinheit | Umsatz | Ausbeute |
|---|---|---|---|
| 2. Methylether | 99,2 % | 95 % | 91 % |
| 3. Ethylether | 98,5 % | 91 % | 92 % |
| 4. n-Butylether | 98,7 % | 89 % | 89 % |
| 5. Methallylether | 97,8 % | 92 % | 87 % |

## Patentansprüche

**1.** N-Oxyl-Derivate des 2,2,6,6-Tetramethylpiperidins der allgemeinen Formel, beinhaltend:

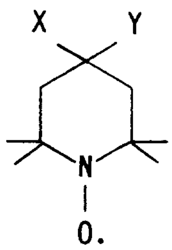

I

1. Ketale, worin

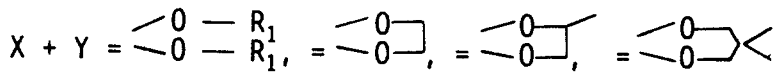

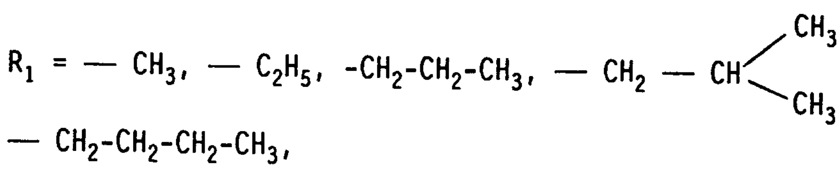

und 2. Ether, worin

Y = —H, X = $OR_2$

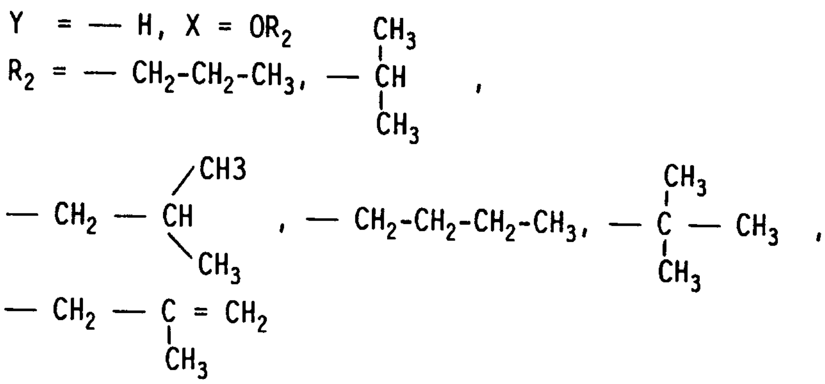

**2.** Verfahren zur Herstellung von neuen N-Oxyl-Derivaten des 2,2,6,6-Tetramethylpiperidins gemäß Anspruch 1, dadurch gekennzeichnet, daß die Oxidation des entsprechenden Derivats des 2,2,6,6-Tetramethylpiperidins der allgemeinen Formel II,

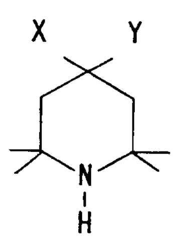

II

mittels Wasserstoffsuperoxid unter der Katalyse von zweiwertigen Metallsalzen erfolgt.

**3.** Verfahren nach Anspruch 2, dadurch gekennzeichnet, daR Salze von Erdalkalimetallen und Zink, insbesondere in Form ihrer Chloride, Sulfate, Nitrate, Phosphate und Hydroxide, eingesetzt werden.

Europäisches Patentamt

# EUROPÄISCHER RECHERCHENBERICHT

Nummer der Anmeldung

EP 93 10 6039

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| A | EP-A-0 389 419 (CIBA-GEIGY AG)<br>* Seite 2 - Seite 4; Abbildung III *<br>--- | 1 | C07D211/94 |
| A | THE JOURNAL OF ORGANIC CHEMISTRY<br>Bd. 52, Nr. 12, 12. Juni 1987, COLUMBUS OHIO<br>Seiten 2559 - 2562<br>PIER LUCIO ANELLI ET AL. 'Fast and Selective Oxidation of Primary Alcohols to Aldehydes.......'<br>* das ganze Dokument *<br>--- | 1 | |
| A | THE JOURNAL OF ORGANIC CHEMISTRY<br>Bd. 55, Nr. 2, 19. Januar 1990, COLUMBUS OHIO<br>Seiten 462 - 466<br>TSUTOMU INOKUCHI ET AL. 'A Selective and Efficient Method for Alcohol Oxidations Mediated by N-Oxoammonium Salts....'<br>* das ganze Dokument *<br>--- | 1 | RECHERCHIERTE SACHGEBIETE (Int. Cl.5 ) |
| A | CHEMICAL ABSTRACTS, vol. 107, no. 21, 23. November 1987, Columbus, Ohio, US;<br>abstract no. 198020a,<br>TROFIMOV, B. A ET AL. 'Vinyl ether of 2,2,6,6-tetramethyl-4-hydroxypiperidine-1-oxyl and its oligomers.'<br>Seite 733 ;Spalte 2 ;<br>* Zusammenfassung *<br>& IZV. AKAD. NAUK SSSR SER. KHIM.<br>Nr. 12, 1986,<br>Seiten 2750 - 2755<br>----- | 1 | C07D |

Der vorliegende Recherchenbericht wurde für alle Patentansprüche erstellt

| Recherchenort | Abschlußdatum der Recherche | Prüfer |
|---|---|---|
| BERLIN | 22 SEPTEMBER 1993 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)